# EUROPEAN PATENT SPECIFICATION

(11) **EP 1 189 851 B1**
(45) Date of publication and mention of the grant of the patent: **20.08.2003**
(21) Application number: 00929681.5
(22) Date of filing: 09.05.2000
(51) Int. Cl.: C04B 35/447, A61L 27/12, A61L 27/32, A61K 6/033

(54) **SINTERED HYDROXYAPATITE COMPOSITIONS AND METHOD FOR THE PREPARATION THEREOF**
GESINTERTE HYDROXYAPATITZUSAMMENSETZUNGEN UND VERFAHREN FÜR DEREN AUFBEREITUNG
COMPOSITIONS HYDROXYAPATITE FRITTEES ET PROCEDE DE PREPARATION DE CELLES-CI

(30) Priority: 11.05.1999 GB 9910928
(43) Date of publication of application: 27.03.2002
(73) Proprietor: University College London, London WC1E 6BT (GB)
(72) Inventor: KNOWLES, Jonathan C., c/o Eastman Dental Institute, London WC1X 8LD (GB); HASTINGS, Garth W., The Institute of Materials, Singapore 119260 (SG); DE SILVA SANTOS, Jose D., Dep. Metallurgical Eng., Oporto (PT)
(74) Representative: Hayes, Adrian Chetwynd
(86) International application number: GB0001766
(87) International publication number: WO00068164

(56) References cited:
- EP-A- 0 577 342
- US-A- 4 149 893
- US-A- 4 708 652

## Description

The present invention relates to hydroxyapatite compositions and, in particular, to sintered composites of hydroxyapatite with a phosphate-based glass.

Hydroxyapatite is a member of the apatite group of minerals and has the chemical formula Ca₁₀(PO₄)₆(OH)₂. It is, essentially, a calcium phosphate including hydroxide having a Ca:P ratio of 1.67:1.

Hydroxyapatite is currently of much interest in the biomedical field due to its well established bioactivity. It has, for example, been used in various dental materials and in various medical applications, such as artificial joints and bones and as bone fillers. The material is, however, limited in its application because of its inadequate mechanical properties and thus has mainly been used for low load applications.

Attempts to enhance the mechanical properties of hydroxyapatite have generally been based upon the development of compaction methods and also the choice and control of grain size. Sintering aids have also previously been suggested for improving the mechanical properties of hydroxyapatite, but these have been single phase materials, such as CuO and Al₂O₃. EP-A-577,342 discloses compositions comprising hydroxyapatite and a biocompatible glass based upon CaO and P₂O₅.

We have now developed sintered hydroxyapatite compositions which are biocompatible and which have enhanced mechanical properties, and also a method for the preparation of these compositions.

Accordingly, the present invention provides a sintered enhanced hydroxyapatite material which comprises hydroxyapatite, a biocompatible glass based upon CaO and P₂O₅ contained in an amount of less than 10% by weight based on the weight of hydroxyapatite and a source of F⁻ ions.

The present invention also provides a method for the preparation of a material as defined above which comprises sintering a mixture of hydroxyapatite, a biocompatible glass based upon CaO and P₂O₅, and a source of F⁻ ions at a temperature of above 1200°C.

The present invention further provides a bone implant or bone filler which comprises a material as defined above.

The present invention also provides a composition such as a bone cement or dental cement which comprises a material as defined above together with a pharmaceutically acceptable diluent or carrier.

The present invention additionally provides a material or composition as defined above for use in a method of treatment of the human or animal body by surgery or therapy.

In the preparation of the composite of the present invention, the biocompatible glass acts as a sintering aid and is contained in an amount of less than 10% by weight, preferably an amount of from 2.5 to 5% by weight, more preferably from 2.5 to 4% by weight, based on the weight of the hydroxyapatite.

By the term "biocompatible glass" as used herein is meant that the glass does not contain any metal ions which are not tolerated by the body (except for Al³⁺, provided that the amount is small).

Suitable biocompatible glasses for use in the present invention are, for example, a two oxide CaO-P₂O₅ glass, a three oxide CaO-P₂O₅-Na₂O glass, or a four oxide CaO-P₂O₅-Na₂O-Al₂O₃. Other components may be incorporated into the biocompatible glass based upon CaO and P₂O₅, provided that the metals of the oxide used in the formation thereof are compatible with the body.

The preferred biocompatible glass for use in the present invention is a two oxide CaO-P₂O₅ glass. The mole ratio of CaO to P₂O₅ in these glasses may vary within wide limits but preferably is from 20:80 to 80:20 mol %. The glass having a mole ratio of CaO:P₂O₅ of about 50:50 is particularly preferred. If CaF₂, or any other compound containing calcium, is used as the source of F⁻ ions, the amount of Ca in this compound is added to the amount of CaO when determining the above ratio. Thus by using such a compound the amount of CaO in the glass may be reduced if appropriate.

The source of F⁻ ions enhances the properties of the material. In particular it improves the strength properties such as the flexural bend strength. The materials disclosed in EP-A-577,342 have a flexural bend strength of up to about 80 to 90 MPa. However, the materials of the present invention have an increased flexural bend strength of, for example, up to about 120 to 160 MPa. This enables the materials to be used in surgical techniques where the materials of EP-A-577,342 could not be used, such as in spinal surgery.

The source of F⁻ ions should not contain any metal ions which are not tolerated by the body. It is, for example, an alkali metal or alkaline earth metal fluoride such as CaF₂, NaF or MgF₂, preferably CaF₂.

The source of F⁻ ions may be contained in the material of the present invention in an amount of up to, for example, 40% by weight, based on the weight of hydroxyapatite. The F⁻ ions themselves may be contained in the material in an amount of up to, for example, 20% by weight, preferably up to 5% by weight, more preferably 1 to 2.5% by weight, most preferably 1.25 to 2% by weight, based on the weight of the hydroxyapatite.

The source of F⁻ ions may be present in the material either separately or incorporated in the biocompatible glass.

The total weight of the glass and source of F⁻ ions is, for example, up to 50% by weight, preferably up to 10% by weight, based on the weight of the hydroxyapatite.

The method for the preparation of the enhanced hydroxyapatite material comprises sintering the aforesaid mixture of hydroxyapatite, biocompatible glass based upon CaO and P₂O₅ and source of F⁻ ions at a temperature of above 1200°C, preferably at a temperature of from 1250° to 1350°C. The source of F⁻ ions may be, for example, incorporated in the biocompatible glass before the sintering. Generally, the mixture to be sintered will be in a very fine particulate form with the particles having a particle size of less than 100 micrometres, preferably less than 75 micrometres, more preferably less than 10 micrometres.

The enhanced hydroxyapatite materials of the present invention have improved mechanical properties as compared to hydroxyapatite without the source of F⁻ ions addition. We believe that the F⁻ ions diffuse into the structure and are interstitial in the composite structure, as indicated by an increase in unit cell dimensions as measured by X-ray diffraction.

The materials of the present invention may also increase osteoblast cell proliferation in vitro, as demonstrated by growing cells directly on the material surface and measuring the number of cells at different times.

The enhanced hydroxyapatites of the present invention may be used in any of the dental and medical applications for which unmodified hydroxyapatite or glass modified hydroxyapatite have previously been used. There may also be additional dental and medical fields in which the hydroxyapatite composites of the present invention may be used which are not open to the unmodified hydroxyapatite. Examples of fields where the enhanced hydroxyapatite materials of the present invention may be used are as bone implants, where an implant of the enhanced hydroxyapatite material may be used, or as bone fillers where a powdered composition may be used as a filling material.

The present invention also includes within its scope a composition which comprises an enhanced hydroxyapatite material as hereinbefore defined together with a pharmaceutically acceptable diluent or carrier. Such compositions find use as bone cements or dental cements. Other applications of the enhanced hydroxyapatite materials of the present invention in the medical and dental field will be readily appreciated by those skilled in the art.

The present invention will be further described with reference to the following Examples.

### Preparation Example 1

Phosphate based glasses were produced from reagent grade chemicals heated at 1300°C in an alumina crucible for 1 hour. The following compositions were prepared, in mole percent.

| | **Glass A** | **Glass B** | **Glass C** | **Glass D** | **Glass E** |
|---|---|---|---|---|---|
| P₂O₅ | 75 | 75 | 75 | 63.75 | 63.75 |
| CaO | 25 | 15 | 20 | 21.25 | 21.25 |
| MgO | 0 | 0 | 0 | 5 | 10 |
| CaF | 0 | 10 | 5 | 10 | 5 |

### Example 1

Each glass was coarsely reduced to a sand type particle and milled in a porcelain ball mill pot for 24 hours. After milling its particle size was less than 100 micrometres and its median particle size, D_{0.5}, was 6 to 9 micrometres according to Laser Diffraction analysis using a Malvern Mastersizer Ms-20. To hydroxyapatite powder each of the glass powders was added, in amounts of 2.5% and 4.0% by weight, with methanol (350 ml of methanol/200g of material) and the powders were wet milled together for a further 24 hours. Following the milling process, the slip was poured and oven dried. The dried powder was then auto sieved to a particle size of less than 75 micrometres. Particle size distribution profiles were carried out after milling.

30mm diameter discs, using 4g of powder, were uniaxially pressed at 288 MPa and fired at a 4°C/min ramp rate up to 1200° to 1350°C, with a one hour hold time at this temperature, followed by natural cooling inside the furnace.

The flexural bending strengths for the compositions are given in Table 1 below. Flexural bending strength measurements were obtained for each sample from a 4-point biaxial bending test in a concentric ring jig with a 20 mm supporting span and a 10 mm loading span at a cross head speed of 5 mm/min on 10 specimens. The concentric ring test eliminates many problems associated with the fracture induced by the edges of the samples although it gives lower results when compared with conventional bending tests. Fracture toughness measurements were performed using an indentation technique proposed by Laugier, J. Mater. Sci., 1987, 6, 355-356, using a 9.8 N load.

The materials were characterized by X-Ray Diffraction (XRD) and their microstructure analysed using SEM. In vitro tests were carried out in a phosphate buffered saline solution and samples were bending strength tested after 1 month.

SEM analysis showed that the hydroxyapatite/glass materials were very dense when sintered at 1250°C, or above.

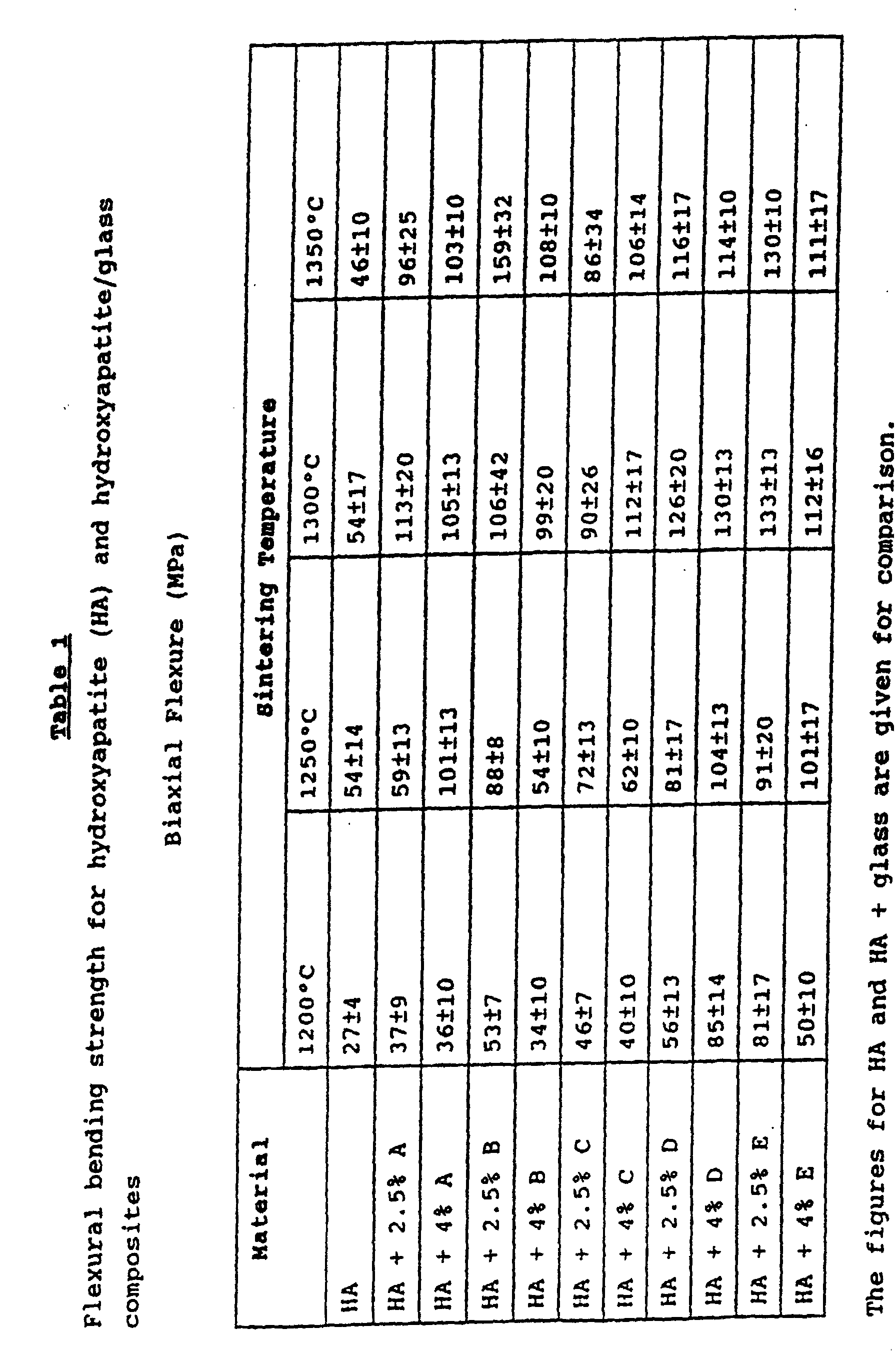

**Table 2**

| Hardness values for the different composites (in kg/mm² or HV) | | | |
|---|---|---|---|
| Composite Composition | 1250°C | 1300° | 1350° |
| HA | 507.77±39.74 | 513.3±51.71 | 498.15±46.67 |
| HA + 2.5% A | 465.75±26.54 | 469.46±59.35 | 534.26±44.82 |
| HA + 4% A | 413.55±40.3 | 512.14±37.84 | 495.69±40.99 |
| HA + 2.5% B | 441.13±35.25 | 593.86±35.04 | 579±45.06 |
| HA + 4% B | 372.04±32.72 | 579.08±28.13 | 535.93±28.04 |
| HA + 2.5% C | 430.09±22.02 | 564.7±27.01 | 565.04±42.85 |
| HA + 4% C | 317.54±32.7 | 536.57±44.49 | 524.84±36.52 |
| HA + 2.5% D | 533.42±30.86 | 545.71±65.36 | 554.5±38.11 |
| HA + 4% D | 555.26±34.34 | 542.11±51.18 | 530.33±8.63 |
| HA + 2.5% E | 544.26±51.66 | 546.02±76.75 | 502.03±46.31 |
| HA + 4% E | 425.48±79.03 | 535.30±33.98 | 474.78±58.81 |

**Table 3**

| Hardness values for the different composites (in kg/mm² or HV) | | | | |
|---|---|---|---|---|
| Composite Composition | 1200°C | 1250°C | 1300°C | 1350°C |
| HA | 85.4±4.4 | 93.1±0.1 | 94±0.3 | 93.9±0.2 |
| HA + 2.5% A | 72.9±0.5 | 93.1±0.9 | 96.3±0.3 | 94.6±0.2 |
| HA + 4% A | 74.6±0.4 | 92.1±0.7 | 97.5±0.7 | 97.2±0.1 |
| HA + 2.5% B | 75.9±0.8 | 89.6±1.4 | 95.1±3.5 | 97.9±0.8 |
| HA + 4% B | 73.9±0.3 | 86.7±0.4 | 97±1.6 | 98±0.8 |
| HA + 2.5% C | 73.9±0.2 | 89.3±0.4 | 95.9±1.7 | 96.9±0.9 |
| HA + 4% C | 76.3±0.4 | 90.2±0.9 | 95.9±1.6 | 95.8±2.2 |
| HA + 2.5% D | 81±0.2 | 96±1 | 96±1.2 | 97.7±0.6 |
| HA + 4% D | 88.1±0.9 | 96±0.2 | 96.2±1.1 | 97.2±0.8 |
| HA + 2.5% E | 87.6±1.1 | 94.7±0.4 | 96.2±0.8 | 95.6±1.6 |
| HA + 4% E | 81.3±0.4 | 97±0.7 | 97.2±0.8 | 98.2±0.3 |

The hardness values are given in Table 2 below. Hardness was measured using Vickers indentation, load 300 g. The percentage of theoretical density measurements are given in Table 3 below. The values in Table 3 are calculated by taking data from a phase analysis and using the values for the theoretical density of the HA, ∝-TCP and β-TCP phases, the theoretical density is calculated from Ritveld. The actual density for the specimens is measured using the Archimedes principle in liquid mercury, and the percentage of theoretical density is calculated.

A considerable improvement in the flexural strength was achieved by the incorporation of the source of F⁻ ions during the sintering of hydroxyapatite.

A slight increase in weight of the samples was detected after immersion in saline for 1 month, which was related to the precipitation of crystals on the surface of the materials. No changes in the flexural strength were found after 1 month.

## Claims

1. A sintered enhanced hydroxyapatite material which comprises hydroxyapatite (Ca₁₀(PO₄)₆(OH)₂), a biocompatible glass based upon CaO and P₂O₅ contained in an amount of less than 10% by weight based on the weight of hydroxyapatite, and a source of F⁻ ions.

2. A material as claimed in claim 1 wherein the source of F⁻ ions is an alkali metal or alkaline earth metal fluoride.

3. A material as claimed in claim 2 wherein the source of F⁻ ions is CaF₂, NaF or MgF₂.

4. A material as claimed in claim 3 wherein the source of F⁻ ions is CaF₂.

5. A material as claimed in any one of the preceding claims wherein the source of F⁻ ions is incorporated in the biocompatible glass.

6. A material as claimed in any one of the preceding claims wherein the F⁻ ions are contained in an amount of up to 5% by weight based on the weight of the hydroxyapatite.

7. A material as claimed in any one of the preceding claims wherein the biocompatible glass is contained in an amount of from 2.5% to 5.0% by weight based on the weight of hydroxyapatite.

8. A material as claimed in any one of the preceding claims wherein the biocompatible glass is a two oxide CaO-P₂O₅ glass.

9. A material as claimed in claim 8 wherein the glass has a mole ratio of CaO:P₂O₅ of about 50:50.

10. A material as claimed in any one of claims 1 to 7 wherein the biocompatible glass is a three oxide CaO-P₂O₅-Na₂O glass.

11. A material as claimed in any one of claims 1 to 7 wherein the biocompatible glass is a four oxide CaO-P₂O₅-Na₂O-Al₂O₃ glass.

12. A method for the preparation of a material as claimed in any one of the preceding claims which comprises sintering a mixture of hydroxyapatite, a biocompatible glass based upon CaO and P₂O₅, and a source of F⁻ ions at a temperature of above 1200°C.

13. A method as claimed in claim 12 wherein the source of F⁻ ions is incorporated in the biocompatible glass before the sintering.

14. A method as claimed in claim 12 or 13 wherein the sintering is carried out at a temperature of from 1250° to 1350°C.

15. A method as claimed in any one of the claims 12 to 14 wherein the mixture of hydroxyapatite, biocompatible glass and source of F⁻ ions has a particle size of less than 75 micrometres.

16. A bone implant or bone filler which comprises a material as claimed in any one of claims 1 to 11.

17. A composition which comprise a material as claimed in any one of claims 1 to 11 together with a pharmaceutically acceptable diluent or carrier.

18. A composition as claimed in claim 17 which is a bone cement or a dental cement.

19. A material as claimed in any one of claims 1 to 11 or a composition as claimed in claim 17 or 18 for use in a method of treatment of the human or animal body by surgery or therapy.

## Patentansprüche

1. Gesintertes verbessertes Hydroxyapatit-Material, das Hydroxyapatit (Ca₁₀(PO₄)₆(OH)₂), ein biokompatibles auf CaO und P₂O₅ basierendes Glas, das in einer Menge von weniger als 10 Gew.-%, bezogen auf das Gewicht von Hydroxyapatit, enthalten ist, und eine Quelle für F⁻-lonen umfasst.

2. Material nach Anspruch 1, wobei die Quelle für F⁻-lonen ein Alkalimetall- oder Erdalkalimetallfluorid ist.

3. Material nach Anspruch 2, wobei die Quelle für F⁻-lonen CaF₂, NaF oder MgF₂ ist.

4. Material nach Anspruch 3, wobei die Quelle für F⁻-lonen CaF₂ ist.

5. Material nach einem der vorhergehenden Ansprüche, wobei die Quelle für F⁻-lonen in das biokompatible Glas inkorporiert ist.

6. Material nach einem der vorhergehenden Ansprüche, wobei die F⁻-lonen in einer Menge von bis zu 5 Gew.-%, bezogen auf das Gewicht des Hydroxyapatits, enthalten sind.

7. Material nach einem der vorhergehenden Ansprüche, wobei das biokompatible Glas in einer Menge von 2,5 Gew.-% bis 5,0 Gew.-%, bezogen auf das Gewicht von Hydroxyapatit, enthalten ist.

8. Material nach einem der vorhergehenden Ansprüche, wobei das biokompatible Glas ein Glas mit zwei Oxiden CaO-P₂O₅ ist.

9. Material nach Anspruch 8, wobei das Glas ein Molverhältnis CaO:P₂O₅ von ungefähr 50:50 aufweist.

10. Material nach einem der Ansprüche 1 bis 7, wobei das biokompatible Glas ein Glas mit drei Oxiden CaO-P₂O₅-Na₂O ist.

11. Material nach einem der Ansprüche 1 bis 7, wobei das biokompatible Glas ein Glas mit vier Oxiden CaO-P₂O₅-Na₂O-Al₂O₃ ist.

12. Verfahren zur Herstellung eines Materials nach einem der vorhergehenden Ansprüche, das das Sintern einer Mischung aus Hydroxyapatit, einem biokompatiblen auf CaO und P₂O₅ basierenden Glas und einer Quelle für F⁻-lonen bei einer Temperatur von über 1200°C umfasst.

13. Verfahren nach Anspruch 12, wobei die Quelle für F⁻-lonen vor dem Sintern in das biokompatible Glas inkorporiert wird.

14. Verfahren nach Anspruch 12 oder 13, wobei das Sintern bei einer Temperatur von 1250°C bis 1350°C durchgeführt wird.

15. Verfahren nach einem der Ansprüche 12 bis 14, wobei die Mischung aus Hydroxyapatit, biokompatiblem Glas und Quelle für F⁻-lonen eine Teilchengröße von weniger als 75 Mikrometer aufweist.

16. Knochenimplantat oder Knochenfüllmittel, das ein Material nach einem der Ansprüche 1 bis 11 umfasst.

17. Zusammensetzung, die ein Material nach einem der Ansprüche 1 bis 11 zusammen mit einem pharmazeutisch annehmbaren Verdünnungsmittel oder Träger umfasst.

18. Zusammensetzung nach Anspruch 17, welche ein Knochenzement oder ein Dentalzement ist.

19. Material nach einem der Ansprüche 1 bis 11 oder Zusammensetzung nach Anspruch 17 oder 18 zur Verwendung in einem Verfahren zur Behandlung des menschlichen oder tierischen Körpers bei einer Operation oder einer Therapie.

## Revendications

1. Matière à base d'hydroxyapatite améliorée frittée, qui comprend de l'hydroxyapatite (Ca₁₀(PO₄)₆(OH)₂), un verre biocompatible à base de CaO et P₂O₅ présent en une quantité inférieure à 10 % en poids sur la base du poids de l'hydroxyapatite, et une source d'ions F⁻.

2. Matière suivant la revendication 1, dans laquelle la source d'ions F⁻ est un fluorure de métal alcalin ou de métal alcalino-terreux.

3. Matière suivant la revendication 2, dans laquelle la source d'ions F⁻ consiste en CaF₂, NaF ou MgF₂.

4. Matière suivant la revendication 3, dans laquelle la source d'ions F⁻ consiste en CaF₂.

5. Matière suivant l'une quelconque des revendications précédentes, dans laquelle la source d'ions F⁻ est incorporée au verre biocompatible.

6. Matière suivant l'une quelconque des revendications précédentes, dans laquelle les ions F⁻ sont présents en une quantité allant jusqu'à 5 % en poids sur la base du poids de l'hydroxyapatite.

7. Matière suivant l'une quelconque des revendications précédentes, dans laquelle le verre biocompatible est présent en une quantité de 2,5 % à 5,0 % en poids sur la base du poids de l'hydroxyapatite.

8. Matière suivant l'une quelconque des revendications précédentes, dans laquelle le verre biocompatible est un verre à deux oxydes CaO-P₂O₅.

9. Matière suivant la revendication 8, dans laquelle le verre a un rapport molaire CaO:P₂O₅ d'environ 50:50.

10. Matière suivant l'une quelconque des revendications 1 à 7, dans laquelle le verre biocompatible est un verre à trois oxydes CaO-P₂O₅-Na₂O.

11. Matière suivant l'une quelconque des revendications 1 à 7, dans laquelle le verre biocompatible est un verre à quatre oxydes CaO-P₂O₅-Na₂O-Al₂O₃.

12. Procédé pour la préparation d'une matière suivant l'une quelconque des revendications précédentes, qui comprend le frittage d'un mélange d'hydroxyapatite, d'un verre biocompatible à base de CaO et P₂O₅, et d'une source d'ions F⁻ à une température supérieure à 1200°C.

13. Procédé suivant la revendication 12, dans lequel la source d'ions F⁻ est incorporée au verre biocompatible avant le frittage.

14. Procédé suivant la revendication 12 ou 13, dans lequel le frittage est effectué à une température de 1250° à 1450°C.

15. Procédé suivant l'une quelconque des revendications 12 à 14, dans lequel le mélange d'hydroxy-apatite, de verre biocompatible et de source d'ions F⁻ a un diamètre de particules inférieur à 75 micromètres.

16. Implant osseux ou charge osseuse, qui comprend une matière suivant l'une quelconque des revendications 1 à 11.

17. Composition qui comprend une matière suivant l'une quelconque des revendications 1 à 11 conjointement avec un diluant ou support pharmaceutiquement acceptable.

18. Composition suivant la revendication 17, qui est un ciment osseux ou un ciment dentaire.

19. Matière suivant l'une quelconque des revendications 1 à 11 ou composition suivant la revendication 17 ou 18, destinée à être utilisée dans une méthode de traitement du corps de l'homme ou d'un animal par chirurgie ou thérapie.
